# FASCICULE DE BREVET EUROPEEN

(11) **EP 3 568 454 B1**
(45) Date de publication et mention de la délivrance du brevet: **17.05.2023**
(21) Numéro de dépôt: 17832790.4
(22) Date de dépôt: 22.12.2017
(51) Int. Cl.: C12G 1/04, C12H 1/00

(54) **PROCÉDÉ ENZYMATIQUE D'ÉLIMINATION DU SULFITE**
ENZYMATISCHES VERFAHREN ZUR ENTFERNUNG VON SULFIT
ENZYMATIC METHOD FOR REMOVING SULPHITE

(30) Priorité: 11.01.2017 FR 1750239
(43) Date de publication de la demande: 20.11.2019
(73) Titulaire: Université de Lorraine, 54052 Nancy Cedex (FR)
(72) Inventeur: LEC, Jean-Christophe, 54500 Vandoeuvre-lès-Nancy (FR); KRIZNIK, Alexandre, 54600 Villers-lès-Nancy (FR); TALFOURNIER, François, 54630 Flavigny-sur-Moselle (FR); BOSCHI, Sandrine, 54850 Mereville (FR)
(74) Mandataire: Novagraaf Technologies
(86) Numéro de dépôt international: PCT/FR2017/053822
(87) Numéro de publication internationale: WO 2018/130760

(56) Documents cités:
- EP-A1- 2 090 647
- WO-A1-2005/107479
- REBECCA JARABAK ET AL: "3-Mercaptopyruvate sulfurtransferase: rapid equilibrium-ordered mechanism with cyanide as the acceptor substrate", BIOCHEMISTRY, vol. 19, no. 5, 4 mars 1980 (1980-03-04), pages 900-904, XP055421634, US ISSN: 0006-2960, DOI: 10.1021/bi00546a012
- YUKA KIMURA ET AL: "Identification of H2S3 and H2S produced by 3-mercaptopyruvate sulfurtransferase in the brain", SCIENTIFIC REPORTS, vol. 5, no. 1, 6 octobre 2015 (2015-10-06) , XP055421566, DOI: 10.1038/srep14774

## Description

### Domaine technique

La présente invention se rapporte à un procédé enzymatique d'élimination du sulfite. En particulier, la présente invention se rapporte à un procédé de transformation du sulfite présent dans une composition en thiosulfate comprenant l'introduction dans ladite composition de 3-mercaptopyruvate sulfurtransférase (3-MST) et d'un substrat de ladite 3-mercaptopyruvate sulfurtransférase.

La présente invention trouve des applications notamment dans le domaine pharmaceutique, cosmétique, agroalimentaire, chimique et tout domaine impliquant l'utilisation de sulfite.

Dans la description ci-dessous, les références entre crochets ([ ]) renvoient à la liste des références présentée à la fin du texte.

### État de la technique

Le sulfite est un composé très utilisé dans l'industrie, car il est peu coûteux et possède un très large spectre d'actions et/ou d'effets. En particulier, le sulfite est utilisé pour ses propriétés antioxydantes, antiseptiques et/ou antioxydasiques.

De par son utilisation dans différents domaine techniques, notamment dans le domaine agroalimentaire, l'effet des sulfites et sa tolérance par l'être humain sont de plus en plus étudiés. En particulier, il a été démontré qu'une consommation excessive de sulfite pouvait entrainer des effets néfastes sur la santé, induire une intolérance voir des effets toxiques.

Une évaluation du sulfite en tant qu'additif alimentaire a été effectuée en 2008 par l'OMS qui a indiqué que l'exposition totale alimentaire aux sulfites diffère entre les pays en raison de différents modes d'utilisation de ceux-ci dans les aliments et de la consommation des dits aliments auxquels les sulfites peuvent être ajoutés. Il a été montré qu'ils étaient présents notamment dans les boissons alcoolisées, notamment le vin, et non alcoolisées, mais aussi dans la charcuterie, notamment les saucisses, dans les fruits secs, dans les noix et les pommes de terre transformées.

Une dose journalière admissible a été déterminée et est de 0 à 0,7 mg/kg de masse corporelle (Organisation Mondiale de la Santé).

La présence de sulfites dans les produits de la vie quotidienne tels que les aliments et les médicaments conduit à l'absorption de quantités journalières importantes susceptibles de provoquer des problèmes de santé tel que maux de têtes, allergies et asthme pour les conséquences les moins graves allant jusqu'à des problèmes cardiovasculaires et une augmentation du risque de cancer, par exemple des tumeurs cérébrales et/ou hépatiques, pour les cas les plus graves (Shih et al., 1977, The New England Journal of Medicine [1]; Andersson et al., 2013, International Archives of Occupational and Environmental Health [2]; Ozsoy et al., 2014, Toxicology and Industrial Health [3]). En outre, les sulfites ont été répertoriés comme faisant partie des dix allergènes prioritaires de l'alimentation (Agence de la santé, gouvernement canadien).

La présence de sulfites dans les produits agroalimentaires peut être désormais considérée comme un problème de santé publique car environ un million de français sont actuellement en surdose (étude réalisée par l'Insee et l'Anses en septembre 2012). Ainsi dans le cadre d'un programme national français (depuis 2009) financé par FranceAgriMer coordonné par l'IFV (Institut Français de la vigne et du Vin) et regroupant plusieurs partenaires techniques tels que l'INRA (Institut National de la Recherche Agronomique), des recherches sont menées depuis de nombreuses années avec pour but de diminuer la quantité finale de sulfites, notamment dans le vin.

Il existe donc un réel besoin de trouver un moyen de réduire la quantité/la présence de sulfite palliant ces défauts, inconvénients et obstacles de l'art antérieur, en particulier un procédé permettant de maîtriser les teneurs en sulfite dans les compositions quel que soit le domaine, notamment alimentaire, pharmaceutique etc.

Il existe dans l'état de la technique des procédés/dispositifs qui sont utilisés /étudiés/ en cours de test afin d'essayer réduire la teneur en sulfite dans des compositions.

Par exemple dans le domaine viticole, une voie explorée est de tester différents itinéraires/procédés de production visant à diminuer l'utilisation du sulfite dans les procédés de vinification, l'objectif à moyen terme étant de diminuer la concentration finale en sulfite entre 50 et 100 mg/L (développé par l'IFV). Une autre voie est d'essayer de substituer le sulfite par un ou plusieurs composé(s), par exemple l'acide ascorbique, le lysozyme ou les acides gras à courte chaîne comme l'acide sorbique. Toutefois, ces différents composés ne permettent pas de reproduire/d'obtenir l'ensemble des effets techniques des sulfites. Ainsi, ils ne permettent pas une substitution efficace, en particulier une substitution à l'identique. En outre, ils peuvent provoquer ou provoquent une modification et altération des propriétés de la composition et notamment des caractères organoleptiques des compositions, notamment du vin (IFV et INRA).

Une autre voie explorée est le retrait/l'élimination des sulfites des compositions, notamment du produit fini après production. Par exemple une stratégie consiste à utiliser une méthode chromatographique permettant de fixer et donc d'éliminer le sulfite présent dans le vin à la fin du procédé (WO2015051187 [4]). Cette technique permet l'élimination des sulfites libres uniquement et pourrait conduire à l'élimination d'autres composants essentiels.

Une autre stratégie consiste à utiliser des enzymes ; par exemple la sulfite oxydase qui transforme le sulfite en sulfate (WO2015051187 [4]). Toutefois, cette réaction est réversible, l'équilibre peut être déplacé et ce procédé est donc susceptible de produire du sulfite à partir de sulfate. En outre, la stabilité de la sulfite oxydase est relative, en particulier l'enzyme n'est pas assez stable pour une utilisation à pH bas comme c'est le cas lorsque le sulfite est ajouté comme additif de conservation. Une autre enzyme, la sulfite réductase qui possède une activité rapide d'élimination du sulfite, a été proposée. Toutefois, le produit obtenu est du sulfure d'hydrogène altérant grandement les propriétés de la composition, et notamment les propriétés organoleptiques (Hermann et al., 2015, Nature [5]).

D'autres procédés d'oxydation des sulfites, par exemple *via* l'ajout de composés et/ou d'enzymes dans la composition ont été également envisagés. Toutefois, ces procédés sont limités de par leur efficacité et/ou l'altération éventuelle des propriétés de la composition, notamment ses propriétés organoleptiques. Un autre document (WO 2005/107479 A1) décrit un procédé d'oxydation du sulfite présent dans une composition, le dit procédé comprenant l'introduction dans ladite composition de chloroplastes. En outre il est connu d'utiliser une composition de 3-mercaptopyruvate sulfurtransférase (3-MST) et d'un substrat (3-mercaptopyruvate) pour dégrader le cyanide en thiocyanate (REBECCA JARABAK ET AL: "3-Mercaptopyruvate sulfurtransferase: rapid equilibrium-ordered mechanism with cyanide as the accepter substrate",BIOCHEMISTRY, vol. 19, no. 5, 4 mars 1980 (1980-03-04), pages 900-904). Enfin, une autre stratégie pour limiter la quantité de sulfites est décrite dans EP2090647 A1. Ce document divulgue un procédé de réduction de l'utilisation de sulfite en réduisant la quantité d'oxygène présente dans du vin à l'aide d'une glucose oxydase.

Il existe donc un réel besoin de trouver un moyen de réduire la quantité/la présence de sulfites, notamment présent dans des compositions, palliant ces défauts, inconvénients et obstacles de l'art antérieur, en particulier un moyen d'éliminer/réduire la quantité de sulfites sans altérations négatives des propriétés de la composition.

### Description de l'invention

La présente invention a précisément pour but de répondre à ce besoin en fournissant une 3-mercaptopyruvate sulfurtransférase (3-MST) qui transforme très efficacement le sulfite en thiosulfate.

En particulier, les inventeurs ont démontré de manière surprenante qu'une 3-mercaptopyruvate sulfurtransférase (3-MST) permet de transformer des sulfites en un autre composé.

Les inventeurs fournissent en particulier un procédé de traitement d'une composition comprenant l'introduction dans ladite composition de 3-mercaptopyruvate sulfurtransférase (3-MST) et au moins un substrat de ladite 3-mercaptopyruvate sulfurtransférase.

En d'autres termes, les inventeurs fournissent un procédé de traitement d'une composition comprenant l'introduction dans ladite composition de 3-mercaptopyruvate sulfurtransférase (3-MST) et au moins un substrat de ladite 3-mercaptopyruvate sulfurtransférase afin de transformer des sulfites présents dans ladite composition.

La présente invention a également pour objet un procédé de transformation du sulfite présent dans une composition comprenant l'introduction dans ladite composition de 3-mercaptopyruvate sulfurtransférase (3-MST) et d'un substrat de ladite 3-mercaptopyruvate sulfurtransférase.

Les inventeurs fournissent également un procédé de transformation du sulfite présent dans une composition en thiosulfate comprenant l'introduction dans ladite composition de 3-mercaptopyruvate sulfurtransférase (3-MST) et d'un substrat de ladite 3-mercaptopyruvate sulfurtransférase.

En particulier, les inventeurs ont démontré de manière surprenante que la 3-mercaptopyruvate sulfurtransférase (3-MST) permet, en présence d'un substrat, de catalyser une réaction transformant le sulfite en thiosulfate.

Les inventeurs ont également démontré de manière surprenante que la transformation du sulfite en thiosulfate par la 3-mercaptopyruvate sulfurtransférase *d'Escherichia coli* (3-MST) est irréversible.

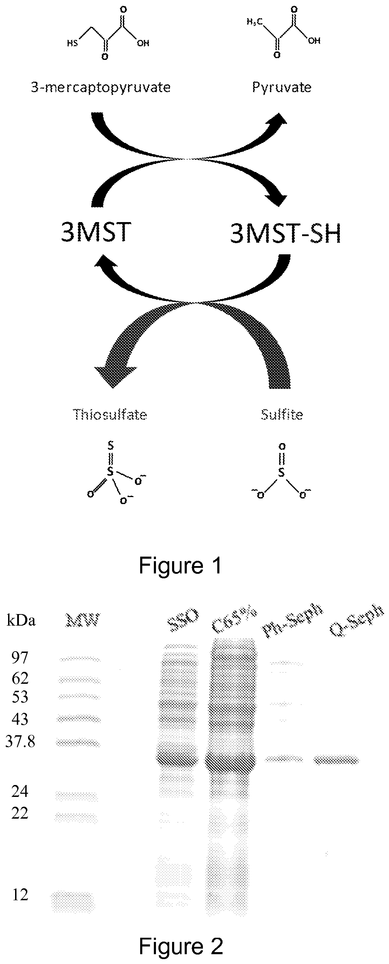

Les inventeurs ont également démontré de manière surprenante que la transformation du sulfite en thiosulfate par la 3-mercaptopyruvate sulfurtransférase (3-MST) est une réaction stoechiométrique irréversible permettant avantageusement de transformer une quantité déterminée de sulfite et/ou la totalité des sulfites présents dans la composition. La figure 1 est un schéma de la réaction enzymatique catalysée par la 3-mercaptopyruvate sulfurtransférase (3-MST) en présence de 3-mercaptopyruvate (3-MP) et de sulfite.

Dans la présente par « traitement d'une composition » on entend l'introduction dans une composition de 3-mercaptopyruvate sulfurtransférase et d'un substrat de ladite 3-mercaptopyruvate sulfurtransférase afin de transformer des sulfites présents en thiosulfates.

Dans la présente par « introduction de la 3-mercaptopyruvate sulfurtransférase et d'un substrat de ladite 3-mercaptopyruvate sulfurtransférase dans la composition » on entend tout moyen d'introduction adapté connu de l'homme du métier de la 3-mercaptopyruvate sulfurtransférase et d'un substrat de ladite 3-mercaptopyruvate sulfurtransférase dans la composition. L'introduction peut être réalisée par versement de la 3-mercaptopyruvate sulfurtransférase et de son substrat dans la composition, par injection, par exemple avec un dispositif de type seringue dans la composition, par aspersion de la composition. L'homme du métier, de par ses connaissances générales saura adapter l'introduction de la 3-mercaptopyruvate sulfurtransférase en fonction de la composition à traiter.

Dans la présente, la 3-mercaptopyruvate sulfurtransférase peut être introduite sous toute forme connue de l'homme du métier adaptée. Par exemple, elle peut être introduite sous la forme de poudre, par exemple lyophilisée, encapsulée ou non ; en solution, par exemple dans du sérum physiologique ou une solution tamponnée.

Dans la présente, le substrat de la 3-mercaptopyruvate sulfurtransférase peut être introduit sous toute forme connue de l'homme du métier adaptée. Par exemple, il peut être introduit sous la forme de poudre, par exemple lyophilisée, encapsulée ou non; en solution, par exemple dans du sérum physiologique ou une solution tamponnée. Dans la présente, la 3-mercaptopyruvate sulfurtransférase peut être une protéine isolée et/ou une protéine recombinante. Il peut s'agir par exemple d'une 3-mercaptopyruvate sulfurtransférase isolée à partir d'un animal, d'un insecte, d'une bactérie, d'un champignon, d'une plante, d'un microorganisme extremophile et/ou d'une Archée. Il peut s'agir d'une 3-mercaptopyruvate sulfurtransférase comprenant dans sa séquence peptidique les trois séquences suivantes :
RXₐWWM (SEQ ID N°13)
CGSGVTAX_{b} (SEQ ID N°14)
GHIX_{c}G (SEQ ID N°15)
dans lesquelles Xₐ est A, V ou L, X_{b} est A ou C, et X_{c} est P ou E.

Les inventeurs ont en effet démontré que les 3-mercaptopyruvate sulfurtransférases comprennent ces séquences en commun dans leur séquence respective et sont capables de catalyser la réaction de transfert de soufre d'un substrat donneur tel que le 3-mercaptopyruvate (3-MP) vers le sulfite en passant par un intermédiaire persulfure transitoire de l'enzyme (Figure 1). La réaction conduit à la formation de pyruvate et de thiosulfate : produits inodores, incolores et non toxiques.

Dans la présente, la 3-mercaptopyruvate sulfurtransférase peut être une enzyme recombinante comprenant une ou plusieurs mutation(s) dans sa séquence peptidique. Il peut s'agir par exemple d'une 3-mercaptopyruvate sulfurtransférase comprenant le remplacement d'au moins un acide aminé acide par un acide aminé hydrophobe. La 3-mercaptopyruvate sulfurtransferase peut être une enzyme recombinante comprenant le remplacement d'un acide aminé acide, par exemple l'acide aspartique ou l'acide glutamique, par un acide aminé comprenant une chaine latérale hydrophobe, par exemple l'alanine, valine, isoleucine, méthionine, phénylalanine, tyrosine, méthionine et/ou tryptophane. Il peut s'agir par exemple d'une 3-mercaptopyruvate sulfurtransférase d'*Escherichia coli* comprenant une substitution en position 17 de sa séquence peptidique de l'acide aspartique par une alanine. Il peut s'agir par exemple de la 3-mercaptopyruvate sulfurtransférase de séquence SEQ ID NO 2, 5, 6 ou 7 du listage de séquences annexé et/ou d'une protéine dont la séquence comprend les peptides de séquences SEQ ID NO 13, 14 et 15.

Les inventeurs ont démontré de manière surprenante que la substitution de l'acide aspartique en position 17 de la 3-mercaptopyruvate sulfurtransférase *d'Escherichia coli* par une alanine permet avantageusement d'augmenter le rendement de production de l'enzyme. Par exemple dans le cas d'une production dans des cellules d'*Escherichia coli,* le rendement de production/purification de ce mutant D17A est supérieur d'un facteur 10 à celui de la forme sauvage de l'enzyme.

Dans la présente invention la 3-mercaptopyruvate sulfurtransférase peut être obtenue à partir d'une séquence nucléotidique codant la 3-mercaptopyruvate sulfurtransférase utilisée dans la présente invention, par exemple les protéines dont la séquence comprend les peptides de séquences SEQ ID NO 13, 14 et 15, le peptide de séquence choisi dans le groupe comprenant les protéines de séquence SEQ ID NO 2, 5, 6 ou 7. Il peut s'agir par exemple d'un acide nucléique comprenant ou constitué de la séquence choisie dans le groupe comprenant la séquence SEQ ID N°1, 8, 9, 10, 11 et 12.

Dans la présente invention, la ou les séquences nucléotidiques codant la 3-mercaptopyruvate sulfurtransférase peu(ven)t être contenue(s) indépendamment dans un vecteur d'expression adapté pour leur expression.

Dans la présente, un vecteur comprenant une séquence nucléotidique codant une des 3-mercaptopyruvate sulfurtransférases utilisées dans la présente invention, par exemple une séquence nucléotidique choisie dans le groupe comprenant les séquences SEQ ID NO 1, 8, 9, 10, 11 et 12. Le vecteur peut être un des vecteurs connus de l'homme du métier pour produire des protéines recombinantes. Il est choisi en général notamment en fonction de l'hôte cellulaire utilisé. Le vecteur peut être par exemple choisi parmi les vecteurs listés dans le catalogue http://www.promega.com/vectors/mammalian_express_vectors.htm [6] ou http://www.qiagen.com/overview/qiagenes.aspx?gaw=PROTQIAgenes080 7&gkw=mammalian+expression [7], ou encore http://www.scbt.com/chap_exp_vectors.php?type=pCruzTM%20Expressio n%20Vectors [8]. Il peut s'agir par exemple du vecteur d'expression décrit dans le document WO 83/004261 [9].

Les acides nucléiques de la présente invention ou les vecteurs de la présente invention sont utilisables notamment pour la production de 3-mercaptopyruvate sulfurtransferase. Aussi, la présente se rapporte également à une cellule hôte comprenant une séquence d'acide nucléique codant pour une des 3-mercaptopyruvate sulfurtransferases ou un vecteur codant pour une des 3-mercaptopyruvate sulfurtransferases.

La cellule hôte ou hôte cellulaire peut être tout hôte approprié pour la production des 3-mercaptopyruvate sulfurtransférases de la présente à partir des vecteurs précités comprenant une séquence nucléotidique codant une 3-mercaptopyruvate sulfurtransferase selon l'invention.

On entend par « cellule hôte » au sens de la présente invention une cellule procaryote ou eucaryote. Des cellules hôtes couramment utilisées pour l'expression de protéines recombinantes incluent notamment des cellules de bactéries telles que *Escherichia coli* ou *Bacillus sp*., des cellules de levures telles que *Saccharomyces cerevisiae,* des cellules de champignons tels que *Aspergillus niger,* des cellules d'insectes, et des cellules de mammifères (notamment humaines) telles que les lignées cellulaires HEK 293, PER-C6, CHO, etc... La transformation des cellules procaryotes et eucaryotes est une technique bien connue de l'homme du métier, par exemple la lipofection, l'électroporation, le choc thermique, ou des méthodes chimiques. En fonction de la cellule à transformer, l'homme du métier pourra aisément déterminer les moyens nécessaires à la transformation de la cellule hôte choisie. Ainsi le vecteur d'expression et la méthode d'introduction du vecteur d'expression au sein de la cellule hôte seront sélectionnés en fonction de la cellule hôte choisie. La cellule hôte transformée par un vecteur d'expression va produire une protéine correspondante par exemple sous forme recombinante. L'homme du métier peut aisément vérifier que la cellule hôte produit la protéine, par exemple recombinante, par exemple en utilisant la technique du Western blot.

Aussi, la présente se rapporte également à un procédé de production de 3-mercaptopyruvate sulfurtransférase par transformation d'une cellule hôte par utilisation d'un vecteur tel que défini ci-dessus et incubation de ladite cellule transformée.

Dans ce procédé, la cellule hôte peut être toute cellule connue de l'homme du métier adaptée. Il peut s'agir par exemple des cellules hôtes telles que définies ci-dessus.

Selon l'invention, la transformation peut être réalisée par tout procédé connu de l'homme du métier. La transformation des cellules procaryotes et eucaryotes est une technique bien connue de l'homme du métier, par exemple la lipofection, l'électroporation, le choc thermique, ou des méthodes chimiques. En fonction de la cellule à transformer, l'homme du métier pourra aisément déterminer les moyens nécessaires à la transformation de la cellule hôte choisie.

Selon l'invention, l'incubation peut être réalisée par tout procédé connu de l'homme du métier. L'incubation/la culture des cellules procaryotes et eucaryotes est une technique bien connue de l'homme du métier. En fonction de la cellule, l'homme du métier pourra aisément déterminer les moyens nécessaires, milieu de culture, conditions de temps et de température requis pour l'incubation/la culture de la cellule hôte choisie.

Selon l'invention, le procédé de fabrication peut comprendre une étape, après transformation et culture/incubation de la cellule transformée, de purification de la 3-mercaptopyruvate sulfurtransférase.

La purification de ladite 3-mercaptopyruvate sulfurtransférase produite peut être réalisée par tout moyen connu de l'homme du métier. Il peut s'agir par exemple d'une technique choisie parmi une électrophorèse, un tamisage moléculaire, une ultracentrifugation, une précipitation différentielle, par exemple au sulfate d'ammonium, une ultrafiltration, une filtration sur membrane ou sur gel, un échange d'ions, une séparation par interactions hydrophobes, ou une chromatographie d'affinité, par exemple de type IMAC.

Dans la présente, la quantité de 3-mercaptopyruvate sulfurtransférase introduite dans la composition peut être comprise entre 100 et 500nM, par exemple de 250 à 500nM. Bien entendu l'homme du métier de par ses connaissances générales saura adapter la quantité de 3-mercaptopyruvate sulfurtransférase à introduire dans la composition.

Dans la présente, le procédé selon l'invention peut comprendre après l'introduction de la 3-mercaptopyruvate sulfurtransférase et d'un substrat de ladite 3-mercaptopyruvate sulfurtransférase une étape d'incubation dans la composition. Dans la présente, l'incubation peut être réalisée pendant un temps de 1 à 60 min, par exemple de 1 à 5 min.

Dans la présente, l'incubation peut être réalisée à une température de 3 à 35°C, par exemple de 4 à 30°C.

Dans la présente, la composition peut être toute composition connue de l'homme du métier. Il peut s'agir par exemple d'une composition chimique, une composition agroalimentaire, une composition pharmaceutique, une composition cosmétique et/ou une composition vétérinaire.

Dans la présente la composition peut être sous toute forme liquide connue de l'homme du métier. Il peut s'agir par exemple d'une solution aqueuse, une solution alcoolique, une solution hydro alcoolique.

Dans la présente la composition agroalimentaire peut être toute composition agroalimentaire, par exemple liquide connue de l'homme du métier susceptible de comprendre des sulfites. Il peut s'agir par exemple de composition alcoolique, par exemple du vin blanc, vin rouge, vin rosé, champagne, bière et/ou cidre, d'aliments par exemples les cornichons, la sauce tomate, le ketchup, la moutarde, la confiture, les fruits en conserve, les jus de fruits, les salades de fruits, les sirops de fruits, les légumes en conserve, le vinaigre, les vinaigrettes et les soupes. Il peut s'agir également d'une composition pour la cuisson d'aliments, par exemple de bouillon.

Dans la présente la composition pharmaceutique peut être toute composition pharmaceutique connue de l'homme du métier susceptible de comprendre des sulfites. Il peut s'agir par exemple d'une composition pour administration intraveineuse, une composition orale, un produit à application cutanée, et/ou toute administration connue de l'homme du métier sous forme de composition/formulation liquide et/ou de compositions sèches hydratables, par exemple une poudre à réhydrater.

Dans la présente la composition vétérinaire peut être toute composition vétérinaire connue de l'homme du métier susceptible de comprendre des sulfites. Il peut s'agir par exemple de toute composition vétérinaire, par exemple liquide, connue de l'homme du métier et/ou de compositions sèches hydratables, par exemple une poudre à réhydrater.

Dans la présente la composition cosmétique peut être toute composition cosmétique connue de l'homme du métier susceptible de comprendre des sulfites. Il peut s'agir par exemple des gels douche, des auto-bronzants.

Dans la présente, par substrat de la 3-mercaptopyruvate sulfurtransférase on entend tout substrat connu de l'homme du métier. Il peut s'agir par exemple du 3-mercaptopyruvate ou de tout composé soufré conduisant à la formation d'un intermédiaire persulfure sur la 3-mercaptopyruvate sulfurtransférase.

Dans la présente, la quantité maximale de substrat, par exemple de 3-mercaptopyruvate, introduit dans la composition peut correspondre à la quantité de sulfite présente dans la composition. Dans la présente, le rapport stoechiométrique dudit substrat et du sulfite présent dans ladite composition peut être au maximum de 1 :1. Par exemple, pour 1L d'une composition contenant 1,25 mmoles de sulfite, la quantité maximale de 3-mercaptopyruvate introduite dans la composition est de 1,25 mmoles. Bien entendu l'homme du métier de par ses connaissances générales saura adapter la quantité de 3-mercaptopyruvate à introduire dans la composition.

Dans la présente, le procédé selon l'invention peut comprendre préalablement à l'introduction dans une composition de 3-mercaptopyruvate sulfurtransférases et/ou de substrats de celles-ci une étape préalable de mesure de la concentration et/ou quantité de sulfites présents dans la composition.

Dans la présente la mesure de la concentration et/ou quantité de sulfites présents dans la composition peut être réalisée par tout procédé adapté connu de l'homme du métier. Il peut s'agir par exemple d'un procédé et/ou dispositif disponible dans le commerce, par exemple le kit SO commercialisé par la société Megazyme International, Irlande (Beutler, H. O. (1988). Sulphite In Methods of Enzymatic Analysis [10]) ou les bandelettes Quantofix Sulfit (marque déposée) commercialisées par la société Macherey-Nagel. La quantité de sulfite peut également être déterminée préalablement à l'application dudit procédé par des méthodes classiques, par exemple utilisées actuellement dans l'industrie vinicole par exemple la méthode de Franz Paul qui est la méthode de référence en oenologie (Bondet Chantal et Raymond Sylvestre, Pratiquer les contrôles en oenologie [11]), et/ou par l'utilisation de kits chimiques, par exemple commercialisés par la société Megazyme International, Irlande.

L'homme du métier, de par ses connaissances générales saura adapter le procédé de mesure de la concentration/quantité de sulfites en fonction de la composition.

Dans la présente, le pourcentage du rapport molaire de 3-mercaptopyruvate introduit dans la composition par rapport aux sulfites présents dans la composition peut être compris de 0,01 à 100 %, par exemple de 25 à 100 %.

Les inventeurs ont démontré que la réaction de transformation de sulfite en thiosulfate présente une stoechiométrie de 1:1 avec la quantité de 3-mercaptopyruvate. Les inventeurs ont également démontré qu'il est possible de transformer une quantité déterminée de sulfite présent dans la composition.

En d'autres termes, le procédé de l'invention permet en outre de contrôler précisément la quantité de sulfites transformés en thiosulfate et également la quantité résiduelle éventuelle présente dans la composition après la mise en oeuvre du procédé.

En outre, le procédé de l'invention permet, lorsque le substrat est le 3-mercaptopyruvate, la production de pyruvate dans la composition. La formation de pyruvate notamment dans des compositions agroalimentaire, pharmaceutique, cosmétique et vétérinaire est sans effet concernant la couleur, l'odeur et/ou une éventuelle toxicité. En d'autres termes le pyruvate et le thiosulfate issus du procédé sont inodores, incolores et non toxiques (rapport sur le thiosulfate de la FDA 223-75-2004, 1975 [12]).

De plus, les produits issus du procédé ne modifient pas négativement les caractéristiques essentielles de la composition, par exemple son aspect, ses propriétés gustatives, ses propriétés olfactives.

Le procédé selon l'invention permet également avantageusement d'obtenir des compositions dont les propriétés organoleptiques sont inchangées. En d'autres termes, le procédé selon l'invention ne modifie pas les propriétés organoleptiques de la composition à laquelle il est appliqué.

De plus, le procédé selon l'invention permet la production de thiosulfate qui avantageusement est utile dans le traitement contre la calciphylaxis (Arrestier et al., 2016, Medicine [13]). En outre, le thiosulfate, de par son effet antioxydant est utile pour la stabilisation de composition alcoolique, par exemple tel qu'utilisé aux Etats-Unis.

Ainsi, l'invention permet avantageusement d'améliorer la stabilité des compositions sur lesquelles elle est utilisée.

D'autres avantages pourront encore apparaître à l'homme du métier à la lecture des exemples ci-dessous, illustrés par les figures annexées, donnés à titre illustratif.

### Brève description des figures

- La figure 1 est un schéma représentant le mécanisme catalytique de la 3-mercaptopyruvate sulfurtransférase dans lequel le 3-mercaptopyruvate est utilisé comme substrat donneur de soufre et le sulfite comme accepteur conduisant à la formation de pyruvate et de thiosulfate.
- La figure 2 est une photographie d'un gel de protéine SDS-PAGE 12,5% montrant les différentes étapes de purification de la 3-mercaptopyruvate sulfurtransférase *d'Escherichia coli.* La colonne MW correspond aux marqueurs de poids moléculaire, la colonne SSO correspond à la fraction soluble obtenue après la lyse des cellules bactériennes, la colonne C65% correspond à la fraction insoluble obtenue après précipitation en présence de 65% de sulfate d'ammonium, la colonne Ph-Seph correspond à la fraction obtenue après chromatographie d'interaction hydrophobe Phenyl-Sépharose (GE Healthcare (marque déposée)) et la colonne Q-Seph correspond à la fraction obtenue après chromatographie échangeuse d'anion Q-Sépharose (GE Healthcare (marque déposée)).

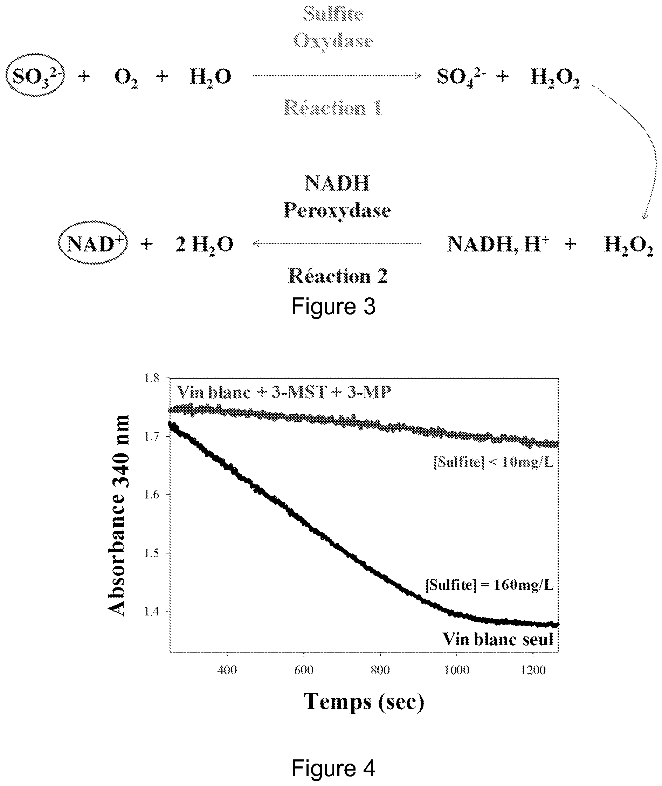

- La figure 3 est un schéma simplifié représentant le principe du kit SO comprenant l'utilisation de deux enzymes chacune catalysant une étape réactionnelle. La première étape fait intervenir la sulfite oxydase (réaction 1) et la deuxième la NADH peroxydase (réaction 2).
- La figure 4 représente le suivi de l'absorbance à 340nm en fonction du temps d'un échantillon de vin blanc non traité (courbe noire vin blanc seul) et d'un échantillon de vin blanc après traitement avec 2 µM de 3-mercaptopyruvate sulfurtransférase et une quantité stoechiométrique (1 :1) de 3-mercaptopyruvate par rapport aux sulfites (courbe supérieure vin blanc+3-MST+3-MP).

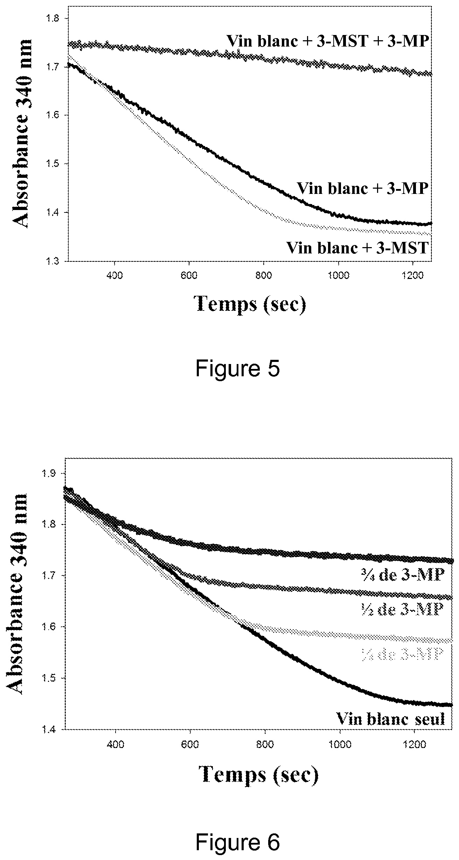

- La figure 5 représente le suivi de l'absorbance à 340nm en fonction du temps d'un échantillon de vin blanc après traitement avec une quantité stoechiométrique (1 :1) de 3-mercaptopyruvate par rapport aux sulfites (courbe noire vin blanc seul), d'un échantillon de vin blanc après traitement avec 2 µM de 3-mercaptopyruvate sulfurtransférase (vin blanc+3-MST) et d'un échantillon de vin blanc après traitement avec 2 µM de 3-mercaptopyruvate sulfurtransférase et une quantité stoechiométrique (1 :1) de 3-mercaptopyruvate par rapport aux sulfites (courbe supérieure vin blanc+3-MST+3-MP).
- La figure 6 représente le suivi de l'absorbance à 340nm en fonction du temps d'un échantillon de vin blanc non traité (courbe noire vin blanc seul), d'échantillons de vins blancs après traitement avec 2 µM de 3-mercaptopyruvate sulfurtransférase et un rapport 3-mercaptopyruvate/sulfite égal à ¼ (courbe ¼ de 3-MP), à ¼ (courbe ½ de 3-MP) et à ¾ (courbe ¾ de 3-MP).

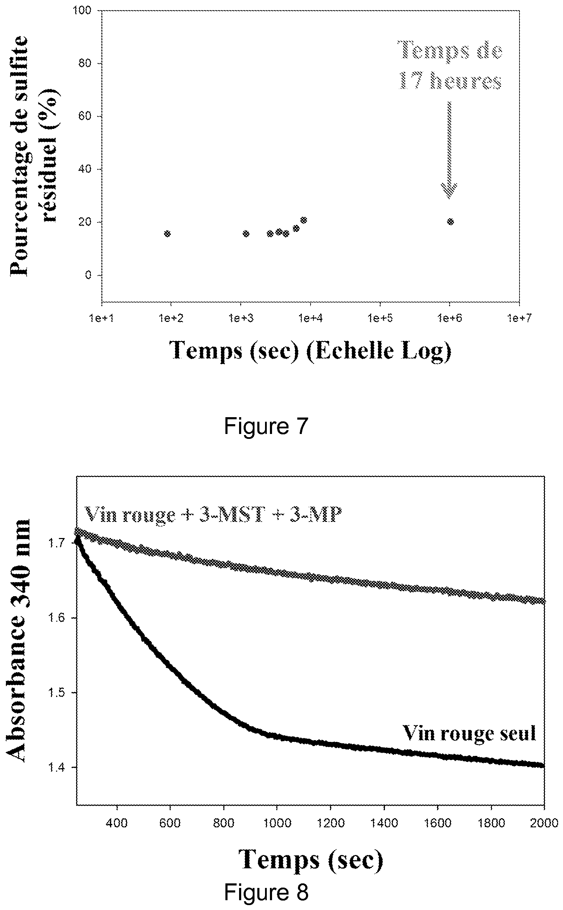

- La figure 7 est un graphique représentant le pourcentage de sulfite résiduel après traitement par ledit procédé (ordonné) en fonction du temps (abscisse).
- La figure 8 représente le suivi de l'absorbance à 340nm en fonction du temps d'un échantillon de vin rouge non traité (courbe noire vin rouge seul) et d'un échantillon de vin rouge après traitement avec 0,5 µM de 3-mercaptopyruvate sulfurtransférase et une quantité stoechiométrique (1 :1) de 3-mercaptopyruvate par rapport aux sulfites (courbe supérieure vin rouge+3-MST+3-MP).

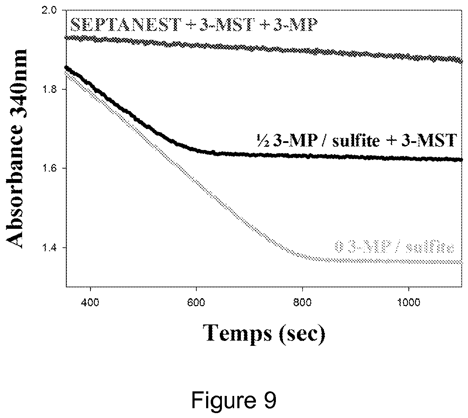

- La figure 9 représente le suivi de l"absorbance à 340nm en fonction du temps d'un anesthésique local (Septanest (marque déposée)) après traitement avec 0,5 µM de 3-mercaptopyruvate sulfurtransférase et du 3-mercaptopyruvate avec un rapport 3-mercaptopyruvate/sulfite égal à 0 (courbe 0 3-MP / sulfite), à ½ (courbe ½ de 3-MP/sulfite) et à 1 (courbe Septanest+3MST+3MP).

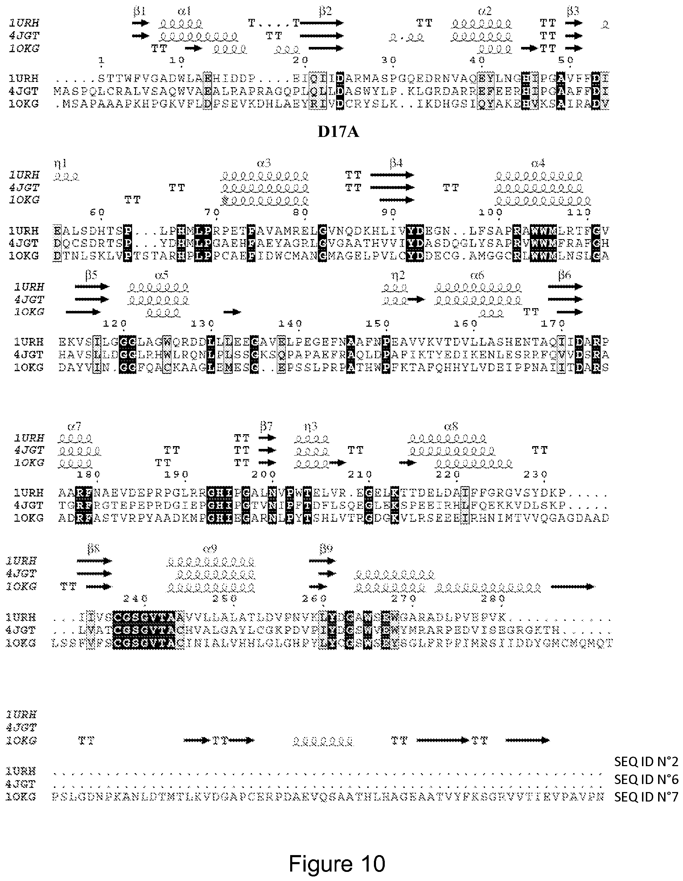

- La figure 10 représente un alignement de séquences protéiques et de structures secondaires de trois 3-mercaptopyruvate sulfurtransférases (3-MST): 3-MST *d'Escherichia coli* (code PDB 1URH), 3-MST humaine (code PDB 4JGT) et 3-MST de *Leishmania major* (code PDB 10KG) mettant en évidence les acides aminés conservés et la position de l'acide aminé (Aspartate 17) qui a été muté en alanine.

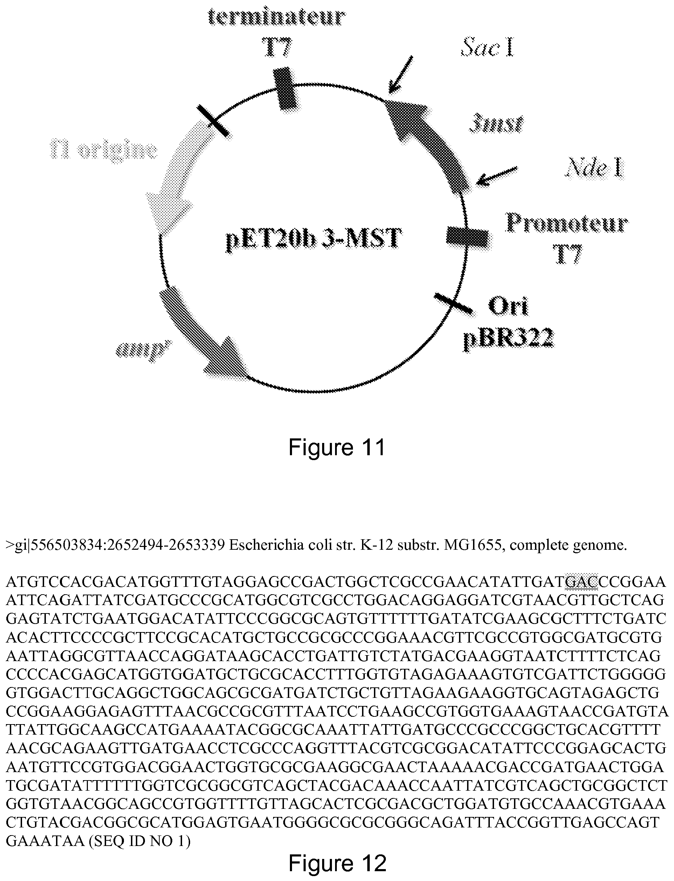

- La figure 11 représente un schéma du vecteur pET20b 3-MST utilisé pour la production de la forme sauvage et du mutant D17A de la 3-mercaptopyruvate sulfurtransférase *d'Escherichia coli.* La construction pET20b 3-MST est un vecteur recombinant obtenu à partir du pET20b commercial Invitrogen (marque déposée).
- La figure 12 correspond à la séquence de la partie codante du gène *3mst* d'*Escherichia coli.* Le codon muté permettant l'obtention du mutant D17A de la 3-mercaptopyruvate sulfurtransférase *d'Escherichia coli* est souligné et surligné en gris.

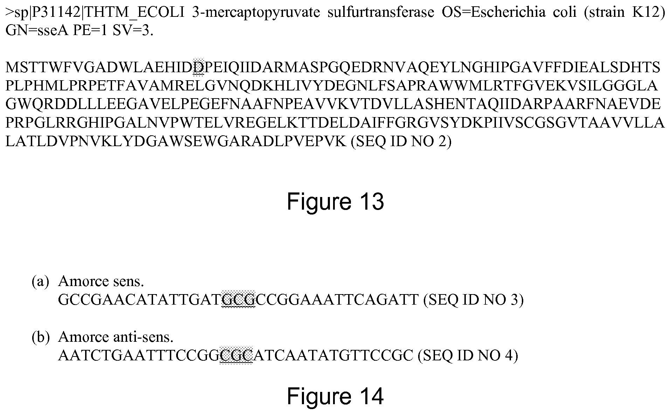

- La figure 13 est la séquence protéique de la forme sauvage de la 3-mercaptopyruvate sulfurtransférase d'*Escherichia coli.* L'acide aspartique souligné et surligné en gris correspond à l'acide aminé muté en alanine dans le mutant D17A de la 3-mercaptopyruvate sulfurtransférase d'*Escherichia coli.*
- La figure 14 contient la séquence des amorces nucléotidiques sens (a) et anti-sens (b) utilisées lors de la mutagenèse dirigée effectuée sur le vecteur d'expression pET20b 3-MST.

### EXEMPLES

### Exemple 1 : Production et purification de la 3-mercaptopyruvate sulfurtransférase D17A d'Escherichia coli

Le plasmide codant la 3-mercaptopyruvate sulfurtransférase D17A *d'Escherichia coli* a été obtenu par mutagenèse dirigée du plasmide codant la forme sauvage de l'enzyme (Figures 11, 12, 13 et 14).

Le mutant D17A de la 3-mercaptopyruvate sulfurtransférase *d'Escherichia coli* a été produit par transformation de bactéries *Escherichia coli* BL21(DE3) avec un vecteur d'expression pET20b-3MST. La transformation de 50 µL de cellules compétentes BL21(DE3) par 100 ng de pET20b-3MST a été réalisée par incubation du mélange dans la glace pendant 30 min suivie d'un choc thermique (30 secondes à 42°C). Le mélange a ensuite été utilisé pour inoculer une préculture de 50 mL de milieu LB supplémenté en ampicilline (200 mg/L). Après incubation d'une nuit à 37°C sous agitation, 4 L de milieu LB supplémenté en ampicilline (200 mg/L) ont été ensemencés au 1/100^{ème} et placées à 37°C sous agitation. Lorsque la densité optique à 600nm a atteint 0,6, l'expression a été induite par l'ajout de 1 mM d'IPTG (Isopropyl-1-thio-β-D-galactopyranoside). Les cellules ont été ensuite placées pendant 3 heures à 37°C sous agitation puis récoltées par centrifugation à 3000 g. Les culots cellulaires ont été repris dans 20 mL de tampon TE (Tris HCl 50mM, EDTA 2mM, pH 8), 10mM de DTT, 20 U/mL de benzonase et 4mM de MgSO₄. La lyse cellulaire a été réalisée par sonication à 4°C sous une puissance de 40W par période de deux fois 2 minutes pendant 50 % du temps. Les débris cellulaires ont été éliminés par centrifugation à 17 000 g pendant 45 minutes.

L'enzyme a été purifiée grâce à un protocole comprenant les 3 étapes suivantes :
- Précipitation au sulfate d'ammonium : La fraction soluble obtenue après sonication a été amenée à 65% de saturation en sulfate d'ammonium (SA) sous légère agitation à 4°C. A cette concentration, la protéine a précipité et après centrifugation à 17 000 g pendant 45 minutes le culot a été alors repris dans 20 mL de tampon TE (Tris HCl 50 mM, EDTA 2 mM, pH 8).
- Chromatographie d'interaction hydrophobe Phenyl-Sepharose (GE Healthcare (marque déposée)) : Du sulfate d'ammonium a été ajouté progressivement à la solution protéique jusqu'à une concentration de 1 M. Cette solution a été alors filtrée (0,2 µm) avant injection sur une colonne d'interaction hydrophobe Phényl Sépharose préalablement équilibrée avec du tampon TE, 1M SA. L'élution est réalisée par gradient décroissant en sulfate d'ammonium (de 1 à 0 M). Les fractions collectées ont été analysées par électrophorèse SDS-PAGE 12,5% et celles contenant la 3-mercaptopyruvate sulfurtransférase ont été rassemblées puis dialysées pendant une nuit contre du tampon TE (Tris HCl 50 mM, EDTA 2 mM, pH 8).
- Chromatographie échangeuse d'anions Q-Sépharose (GE Healthcare (marque déposée)): Après dialyse, la solution protéique a été filtrée à 0,2 µm puis injectée sur une colonne de chromatographie échangeuse d'anions Q-Sépharose préalablement équilibrée avec du tampon TE (GE Healthcare (marque déposée)). L'élution a été réalisée en appliquant un gradient croissant de KCl (0 à 1 M). Les fractions contenant la 3-mercaptopyruvate sulfurtransférase ont été identifiées par électrophorèse SDS-PAGE 12,5% puis rassemblées et concentrées par ultrafiltration sur cellule Amicon en utilisant une membrane YM 10 (seuil de 10 kDa). La solution protéique ainsi purifiée a été amenée à une saturation de 70 % en sulfate d'ammonium afin de précipiter la protéine et la conserver à -20°C en présence de 5 mM de DTT.

La figure 2 est une photographie d'un gel de protéine SDS-PAGE 12,5% après migration des échantillons obtenus après chacune des étapes de purification de la 3-mercaptopyruvate sulfurtransférase d'*Escherichia coli.* Cette figure illustre clairement les différentes étapes de purifications démontrant l'obtention de la 3-mercaptopyruvate sulfurtransférase purifiée à homogénéité.

### Exemple 2 : Elimination du sulfite présent dans du vin blanc

La 3-mercaptopyruvate sulfurtransférase utilisée était le mutant D17A de la 3-mercaptopyruvate sulfurtransférase d'*Escherichia coli* purifiée obtenue dans l'exemple 1 ci-dessus.

L'étude a été réalisée avec un vin blanc sec (Colombard Sauvignon, Côte de Gascogne, 44330 La chapelle Heulin, France). La concentration en sulfite dans ce vin a été mesurée à 160 mg/L avant traitement par l'utilisation du kit sulfite oxydase (kit SO) (Megazyme International, Irlande) (courbe en noir, figure 4). La quantité en sulfite est donnée par la différence de Densité Optique à 340 nm (ΔDO340nm) mesurée à l'aide d'un spectrophotomètre (SAFAS UVmc²).

La densité optique a été mesurée à 340 nm car elle permet de suivre la quantité de NADH oxydé en NAD⁺ (réaction 2, figure 3) qui est corrélée à la quantité de sulfite transformée en sulfate (réaction 1, figure 3). En d'autres termes, la diminution de la densité optique correspond à l'oxydation des sulfites par la sulfite oxydase démontrant ainsi la présence de sulfite.

Le kit SO permet de doser la quantité des sulfites totaux du vin c'est-à-dire les sulfites libres et les sulfites combinés aux molécules organiques. Ledit procédé présente l'avantage d'éliminer les sulfites totaux, or les formes combinées une fois dans l'estomac à pH de 1,5 à 5 sont libérées et constituent une source non négligeable de sulfite dans l'organisme qu'il est nécessaire de supprimer. Si la méthode permet d'éliminer les sulfites totaux, c'est que l'élimination des formes sulfite libres conduit à un déplacement de l'équilibre chimique vers la décombinaison des sulfites fixés et donc *in fine* à l'élimination totale des sulfites (Grignard et al., 1950, Traité de chimie organique [14]).

Afin de déterminer si la 3-mercaptopyruvate sulfurtransférase permet effectivement de transformer le sulfite en thiosulfate, 25 mL de vin blanc ont été mélangés avec 2 µM de 3-mercaptopyruvate sulfurtransférase et du 3-mercaptopyruvate en stoechiométrie 1:1 par rapport au sulfite. L'ensemble a été incubé 5 min à température 25°C et la densité optique à 340 nm mesurée. La figure 4 présente les résultats obtenus.

La densité optique a été mesurée et il n'y a plus de diminution de l'absorbance attestant d'une élimination totale du sulfite présent dans le vin démontrant la transformation du sulfite en thiosulfate (courbe Vin blanc+3-MST+3MP, figure 4). Différents témoins montrent que l'élimination du sulfite est bien liée à l'activité enzymatique de l'enzyme puisque la même expérience réalisée en absence du substrat 3-mercaptopyruvate ou en absence d'enzyme n'entraîne pas de baisse de la quantité de sulfite dans le vin. Les résultats obtenus sont représentés sur la figure 5 (respectivement courbes Vin blanc+3-MST et Vin blanc + 3MP).

L'élimination des sulfites libres dans le vin blanc a également été mise en évidence par l'utilisation de bandelettes (Quantofix (marque déposée)) Sulfit, Macherey-Nagel) (résultats non divulgués).

Par contre la quantité de sulfite après le procédé n'a pas pu être déterminée par les méthodes classiques utilisées actuellement dans l'industrie vinicole telles que :
a. La méthode de Franz Paul car elle nécessite une acidification forte et agressive par ajout d'acide sulfurique et chauffage ce qui conduit dans ces conditions à la dismutation du thiosulfate en sulfite,
b. L'utilisation de kits chimiques car ils contiennent des réactifs spécifiques des groupements thiols or le thiosulfate et l'enzyme possèdent des groupements thiolates constituant une source de réactions « parasites » biaisant la mesure.

Le dosage de sulfite après la mise en oeuvre d'un exemple de procédé conforme à la présente invention a été fait par l'utilisation du kit SO (Megazyme International, Irlande) qui constitue une méthode de dosage spécifique quantitative et par l'utilisation de bandelettes (Quantofix (marque déposée)) Sulfit, Macherey-Nagel) permettant de valider cette méthode en tant que test qualitatif.

Tel que mentionné ci-dessus, le kit SO permet de doser la quantité des sulfites totaux du vin c'est-à-dire les sulfites libres et les sulfites combinés aux molécules organiques. Ce dosage a été réalisé selon le protocole préconisé dans une cuve en verre en mélangeant 1,3 mL d'eau distillée, 250 µL de tampon, 100 µL d'échantillon étudié, 100 µL de NADH, 10 µL de NADH peroxydase. Après stabilisation de l'absorbance, 10 µL de sulfite oxydase ont été ajoutés et la variation d'absorbance à 340 nm a été mesurée.

Afin de déterminer la concentration optimale de 3-mercaptopyruvate sulfurtransférase à utiliser, des essais ont été réalisés en présence de 3-mercaptopyruvate en quantité stoechiométrique par rapport aux sulfites et de concentrations variables de 3-mercaptopyruvate sulfurtransférase. La densité optique à 340 nm a été mesurée instantanément suite au mélangé réactionnel.

La concentration de sulfite dans l'échantillon de vin blanc était de 160 mg/L. Une mesure de la concentration résiduelle de sulfite après mise en oeuvre du procédé a été effectuée à l'aide du kit SO. Les résultats obtenus en présence de différentes concentrations de 3-mercaptopyruvate sulfurtransférase sont présentés dans le tableau 1 ci-dessous et sur la figure 5.

**Tableau 1 : Quantité de sulfite résiduelle en fonction de la concentration de 3-mercaptopyruvate sulfurtransférase**

| | | | | | |
|---|---|---|---|---|---|
| Concentration d'enzyme (3-MST) en nM | 100 | 250 | 350 | 450 | 500 |
| Concentration de sulfite résiduelle (mg/L) | 160 | 96 | 50 | 25 | <10 |

Tel que démontré ci-dessus, la 3-mercaptopyruvate sulfurtransférase permet d'éliminer les sulfites présents dans le vin blanc et une concentration de 500 nM d'enzyme permet une élimination de l'ensemble des sulfites présents.

En complément, des essais en présence de concentrations variables en 3-mercaptopyruvate et de 500 nM de 3-mercaptopyruvate sulfurtransférase ont été réalisés sur un échantillon de vin blanc. En particulier des ratios molaires 3-mercaptopyruvate/sulfite de ¼, ½ et ¾ ont été testés. Une mesure de la concentration résiduelle de sulfite après mise en oeuvre du procédé a été effectuée à l'aide du kit SO. Les résultats obtenus en présence de différentes concentrations de 3-mercaptopyruvate sont présentés dans le tableau 2 ci-dessous et sur la figure 6.

**Tableau 2 : Quantité de sulfite résiduelle en fonction de la concentration de 3-mercaptopyruvate**

| | | | | | | |
|---|---|---|---|---|---|---|
| Pourcentage de 3-mercaptopyruvate / sulfite (mol/mol) | 0 | 25 | 50 | 75 | 95 | 100 |
| Concentration de sulfite résiduelle (mg/L) | 168 | 112 | 86 | 46 | 13,4 | <10 |

Tel que démontré dans le tableau 2 ci-dessus et dans la figure 6, le procédé selon l'invention permet également avantageusement de maitriser la concentration finale de sulfite.

En d'autres termes, le procédé selon l'invention permet avantageusement de modifier la concentration de sulfite dans une composition / échantillon à une valeur déterminée ou de supprimer/transformer les sulfites présents dans une composition, par exemple du vin.

Enfin, le thiosulfate est une molécule stable chimiquement. Une vérification de sa stabilité dans le vin a été effectuée. Pour cela, la mesure de la quantité résiduelle de sulfite après traitement a été réalisée en fonction du temps selon le procédé suivant : Une fois le procédé d'élimination appliqué sur un volume de 25 mL de vin selon le même protocole que décrit ci-dessus (500 nM 3-mercaptopyruvate sulfurtransférase, avec une quantité stoechiométrique de 3-mercaptopyruvate / sulfite), l'échantillon a été stocké à température ambiante pendant 2 semaines et une quantification de la quantité de sulfite résiduelle a été réalisée à intervalles de temps réguliers, à savoir tous les trois jours. Les résultats obtenus ont montré que sur un temps de 17 h (ou deux semaines, résultats non montrés), il n'y a pas libération de sulfite par dismutation du thiosulfate dans le vin (figure 7).

En d'autres termes, un procédé conforme à la présente permet d'éliminer les sulfites en les modifiant en thiosulfates dans une composition, la modification étant stable dans le temps.

### Exemple 3 : élimination du sulfite dans le vin rouge

Dans le présent exemple, la composition était du vin rouge (Corbières, France). Les tanins présents dans le vin rouge interférant avec le procédé de quantification du sulfite (kit SO précité), il a été nécessaire de les éliminer afin de démontrer la disparition du sulfite dans la composition.

Le procédé d'élimination des sulfites a été réalisé sur le vin rouge selon le protocole suivant : 500 nM de 3-mercaptopyruvate sulfurtransférase, quantité stoechiométrique de 3-mercaptopyruvate / sulfite présent dans ce vin. L'élimination des tanins a été effectuée par précipitation en présence de polyvinylpolypyrollidone (PVPP) selon le procédé suivant : 10 mL d'échantillon de vin rouge ont été traités avec 0,2 g de PVPP et une centrifugation à 4000 g a été réalisée pendant 5 minutes. Cette étape est répétée 3 fois.

Une mesure de la concentration de sulfite avant et après mise en oeuvre du procédé a été effectuée à l'aide du kit SO selon le même protocole que décrit dans l'exemple 1. Les résultats obtenus sont présentés dans la figure 8. La concentration de sulfite dans le vin rouge était de 100 mg/L (courbe noire vin rouge seul). L'ajout de 500 nM de 3-mercaptopyruvate sulfurtransférase et d'une quantité stoechiométrique de 3-mercaptopyruvate a permis l'élimination du sulfite (courbe supérieure vin rouge+3-MST+3-MP).

Cet exemple démontre clairement qu'un procédé selon l'invention permet d'éliminer les sulfites présents dans du vin rouge.

### Exemple 4 : élimination du sulfite dans un anesthésique

Dans cet exemple, la composition était un anesthésique local (SEPTANEST (marque déposée)) 40 mg/mL, Adrénalinée au 1/100 000, Chl. articaïne, 40 mg/mL, Laboratoire Septodont).

Le procédé d'élimination des sulfites de la composition a été réalisé selon le protocole suivant : 500 nM de 3-mercaptopyruvate sulfurtransférase, quantité stoechiométrique de 3-mercaptopyruvate / sulfite présent dans la composition.

Une mesure de la concentration de sulfite avant et après mise en oeuvre du procédé a été effectuée à l'aide du kit SO selon le même protocole que décrit dans l'exemple 1. Les résultats obtenus sont présentés dans la figure 9. La concentration de sulfite dans la composition était de 410 mg/L (courbe 0 3-MP / sulfite). L'ajout de 500 nM de 3-mercaptopyruvate sulfurtransférase et d'une quantité stoechiométrique de 3-mercaptopyruvate a permis l'élimination totale du sulfite présent dans la composition (courbe Septanest+3MST+3MP) en accord avec la présente invention.

En outre, le procédé a été mis en oeuvre avec un ratio ¼ de 3-mercaptopyruvate /sulfite (mol/mol) (figure 9, courbe ½ de 3-MP/sulfite). Tel que démontré la moitié des sulfites présents dans la composition sont transformés en thiosulfate.

Cet exemple démontre donc clairement qu'un procédé en accord avec la présente invention permet de traiter des compositions pharmaceutiques afin réduire la quantité de sulfite présent.

### Liste de références

1. Shih et al., 1977, The New England Journal of Medicine.
2. Andersson et al., 2013, International Archives of Occupational and Environmental Health.
3. Ozsoy et al., 2014, Toxicology and Industrial Health.
4. WO2015051187.
5. Hermann et al., 2015, « The octahaem MccA is a haem c-copper sulfite reductase" Nature 520, 706-709
6. http://www.promega.com/vectors/mammalian_express_vectors.htm.
7. http://www.qiagen.com/overview/qiagenes.aspx?gaw=PROTQIAgen es0807&gkw=mammalian+expression
8. http://www.scbt.com/chap_exp_vectors.php?type=pCruzTM%20Exp ression%20Vectors.
9. WO 83/004261
10. Beutler, H. O., 1988, Sulphite In Methods of Enzymatic Analysis.
11. Bondet et Sylvestre, 2005, Pratiquer les contrôles en oenologie.
12. Rapport de la FDA concernant le thiosulfate de sodium, SCOGS-52, 1975.
13.Arrestier et al., 2016, Medicine, 95 (6).
14.Grignard et al., 1950, Traité de chimie organique, 769-770.

## Revendications

1. Procédé de traitement d'une composition comprenant des sulfites, ledit procédé comprenant l'introduction dans ladite composition de 3-mercaptopyruvate sulfurtransférase (3-MST) et d'un substrat de ladite 3-mercaptopyruvate sulfurtransférase afin de transformer lesdits sulfites.

2. Procédé selon la revendication 1 dans lequel lesdits sulfites sont transformés en thiosulfate.

3. Procédé selon la revendication 1 ou 2 dans lequel ladite 3-mercaptopyruvate sulfurtransférase est recombinante.

4. Procédé selon la revendication 1 ou 2 dans lequel ladite 3-mercaptopyruvate sulfurtransférase comprenant dans sa séquence peptidique les trois séquences suivantes :
RXₐVWVM (SEQ ID N°13)
CGSGVTAX_{b} (SEQ ID N°14)
GHIX_{c}G (SEQ ID N°15)
dans lesquelles Xₐ est A, V ou L, X_{b} est A ou C, et X_{c} est P ou E.

5. Procédé selon l'une quelconque des revendications 1 à 3 dans lequel ladite 3-mercaptopyruvate sulfurtransférase est *d'Escherichia coli.*

6. Procédé selon l'une quelconque des revendications 1 à 4 dans lequel la 3-mercaptopyruvate sulfurtransférase est une protéine de séquence peptidique choisi dans le groupe comprenant la séquence SEQ ID N°2, 5, 6 et 7.

7. Procédé selon l'une quelconque des revendications 1 à 5 dans lequel la composition est une composition agroalimentaire, pharmaceutique, cosmétique, vétérinaire.

8. Procédé selon l'une quelconque des revendications 1 à 6 dans lequel ledit substrat de ladite 3-mercaptopyruvate sulfurtransférase est choisi dans le groupe comprenant le 3-mercaptopyruvate.

9. Procédé selon l'une quelconque des revendications précédentes dans lequel le rapport stoechiométrique dudit substrat et du sulfite présent dans ladite composition est au maximum de 1 :1.

## Patentansprüche

1. Verfahren zur Behandlung einer Zusammensetzung, die Sulfite enthält, wobei das Verfahren das Einführen von 3-Mercaptopyruvat-Sulfurtransferase (3-MST) und eines Substrats der 3-Mercaptopyruvat-Sulfurtransferase in die Zusammensetzung umfasst, um die Sulfite umzuwandeln.

2. Verfahren nach Anspruch 1, wobei die Sulfite in Thiosulfat umgewandelt werden.

3. Verfahren nach Anspruch 1 oder 2, wobei die 3-Mercaptopyruvat-Sulfurtransferase rekombinant ist.

4. Verfahren nach Anspruch 1 oder 2, wobei die 3-Mercaptopyruvat-Sulfurtransferase in ihrer Peptidsequenz die folgenden drei Sequenzen umfasst:
RXaWWM (SEQ ID No. 13)
CGSGVTAXb (SEQ ID No. 14)
GHIXcG (SEQ ID No. 15)
worin Xa A, V oder L ist, Xb A oder C ist und Xc P oder E ist.

5. Verfahren nach einem der Ansprüche 1 bis 3, wobei die 3-Mercaptopyruvat-Sulfurtransferase aus Escherichia coli stammt.

6. Verfahren nach einem der Ansprüche 1 bis 4, wobei die 3-Mercaptopyruvat-Sulfurtransferase ein Protein mit einer Peptidsequenz ist, die aus der Gruppe bestehend aus SEQ ID NOs: 2, 5, 6 und 7 ausgewählt ist.

7. Verfahren nach einem der Ansprüche 1 bis 5, wobei die Zusammensetzung eine landwirtschaftliche, pharmazeutische, kosmetische oder tierärztliche Zusammensetzung ist.

8. Verfahren nach einem der Ansprüche 1 bis 6, wobei das Substrat der 3-Mercaptopyruvat-Sulfurtransferase ausgewählt ist aus der Gruppe, die das 3-Mercaptopyruvat.

9. Verfahren nach einem der vorhergehenden Ansprüche, wobei das stöchiometrische Verhältnis des Substrats und des in der Zusammensetzung vorhandenen Sulfits höchstens 1:1 beträgt.

## Claims

1. A method for treating a composition comprising sulfites, said method comprising introducing into said composition 3-mercaptopyruvate sulfurtransferase (3-MST) and a substrate of said 3-mercaptopyruvate sulfurtransferase to transform said sulfites.

2. The method according to claim 1 wherein said sulfites are converted to thiosulfate .

3. The method according to claim 1 or 2 wherein said 3-mercaptopyruvate sulfurtransferase is recombinant.

4. The method according to claim 1 or 2 wherein said 3-mercaptopyruvate sulfurtransferase comprising in its peptide sequence the following three sequences:
RXaWWM (SEQ ID No. 13)
CGSGVTAXb (SEQ ID No. 14)
GHIXcG (SEQ ID No. 15)
wherein Xa is A, V or L, Xb is A or C, and Xc is P or E.

5. The method according to any one of claims 1 to 3 wherein said 3-mercaptopyruvate sulfurtransferase is from Escherichia coli.

6. The method according to any one of claims 1 to 4 wherein the 3-mercaptopyruvate sulfurtransferase is a protein of peptide sequence selected from the group consisting of SEQ ID NOs: 2, 5, 6 and 7.

7. The method according to any one of claims 1 to 5 wherein the composition is an agri-food, pharmaceutical, cosmetic, veterinary composition.

8. The method according to any one of claims 1 to 6 wherein said substrate of said 3-mercaptopyruvate sulfurtransferase is selected from the group comprising the 3-mercaptopyruvate.

9. The method according to any one of the preceding claims wherein the stoichiometric ratio of said substrate and sulfite present in said composition is at most 1:1.
